# EUROPEAN PATENT APPLICATION

(11) **EP 3 260 037 A1**
(43) Date of publication of application: **27.12.2017**
(21) Application number: 16772078.8
(22) Date of filing: 26.02.2016
(51) Int. Cl.: A61B 1/06, A61B 1/00

(54) **ILLUMINATION LIGHT TRANSMISSION DEVICE AND ILLUMINATION LIGHT TRANSMISSION METHOD**

(30) Priority: 31.03.2015 JP 2015070888
(71) Applicant: Sony Corporation, Tokyo 108-0075 (JP)
(72) Inventor: NAKAO, Isamu, Tokyo 108-0075 (JP); TANAKA, Eiichi, Tokyo 108-0075 (JP); FURUKAWA, Akio, Tokyo 108-0075 (JP)
(74) Representative: D Young & Co LLP
(86) International application number: PCT/JP2016/056690
(87) International publication number: WO 2016/158194

(57) **Abstract**

A technology is provided that is capable of reducing the size of an apparatus and improving the work environment of surgical operations as compared with those of related-art technologies. The present technology provides an illumination light transmission apparatus having a non-speckle illumination light source configured to radiate a non-speckle illumination light; a speckle illumination light source configured to radiate a speckle illumination light; a fiber bundle configured by a plurality of optical fibers into a bundle; and an optical control block configured to guide at least one of the non-speckle illumination light and the speckle illumination light into the fiber bundle.

## Description

### [Technical Field]

The present disclosure relates to an illumination light transmission apparatus and an illumination light transmission method. More particularly, the present disclosure relates to an illumination light transmission apparatus and an illumination light transmission method that use a speckle illumination light emitted from a speckle illumination light source.

### [Background Art]

Conventionally, a blood-vessel and blood-flow angiography based on the time change of speckle patterns for the application to surgical operations using endoscopes or microscopes (refer to PTL 1 below). It should be noted that, in what follows, the images obtained by the above-mentioned angiography are referred to as speckle blood-flow images.

In addition, a technology that allows the simultaneous observation of a speckle blood-flow image and an ordinary bright-field image through a surgical microscope is known (refer to NPL 1 below).

### [Citation List]

### [Patent Literature]

[PTL 1]
U.S. Patent No. 6,944,494

### [Non Patent Literature]

[NPL 1]
Neurophotonics 1(1), 015006 (Jul-Sep 2014) pp. 1-6

### [Summary]

### [Technical Problem]

Since, with the technology cited in NPL 1 mentioned above, an illumination optical system has two systems of illumination optical paths between a bright-field image and a speckle blood-flow image, a large housing of an observation apparatus is required, eventually resulting in a problem of deteriorating the work environment for surgical operations.

Therefore, the main object of the present disclosure is to downsize the apparatus so as to improve the work environment for surgical operations as compared with those of the related-art technologies.

### [Solution to Problem]

Earnest researches by the inventors of the present application so as to solve the object mentioned above have resulted in the completion of the present invention by use of speckles that are noises to a non-speckle illumination light and by combinations of a non-speckle illumination light and a speckle illumination light.

That is, the present disclosure first provides an illumination light transmission apparatus. This illumination light transmission apparatus includes a non-speckle illumination light source configured to radiate a non-speckle illumination light, a speckle illumination light source configured to radiate a speckle illumination light, a fiber bundle configured by a plurality of optical fibers into a bundle, and an optical control block configured to guide at least one of the non-speckle illumination light and the speckle illumination light into the fiber bundle.

The optical fibers configuring the fiber bundle of the illumination light transmission apparatus related with the present disclosure may include a single-mode optical fiber.

The optical control block of the illumination light transmission apparatus related with the present disclosure may guide the speckle illumination light into only one optical fiber selected from the optical fibers configuring the fiber bundle.

For the one optical fiber selected from among the optical fibers configuring the fiber bundle of the illumination light transmission apparatus related with the present disclosure, a single-mode optical fiber may be mentioned.

For the one optical fiber selected from among the optical fibers configuring the fiber bundle of the illumination light transmission apparatus related with the present disclosure, an optical fiber in which a polarized-wave face is maintained may be mentioned.

The optical control block of the illumination light transmission apparatus related with the present disclosure may selectively guide the non-speckle illumination light and the speckle illumination light into the fiber bundle.

The optical control block of the illumination light transmission apparatus related with the present disclosure may guide the speckle illumination light into the one selected optical fiber on the basis of an illuminance distribution of the fiber bundle. The illumination light transmission apparatus related with the present disclosure may further include a diffusion plate arranged on an optical path of the non-speckle illumination light so as to diffuse the non-speckle illumination light.

The non-speckle illumination light source of the illumination light transmission apparatus related with the present disclosure may be used for obtaining a bright-field image and the speckle illumination light source may be used for obtaining a speckle-enhanced image.

In the illumination light transmission apparatus related with the present disclosure, the speckle-enhanced image may be at least one of images of a fluid and a flow path.

In the illumination light transmission apparatus related with the present disclosure, for the bright-field image, an organ image may be mentioned and, for the speckle-enhanced image, a blood-flow image may be mentioned.

Then, the present disclosure provides an illumination light transmission method. This illumination light transmission method includes a non-speckle illumination light radiation process of radiating a non-speckle illumination light for use in obtaining a bright-field image, a speckle illumination light radiation process of radiating a speckle illumination light for use in obtaining a speckle-enhanced image, and an emission process of emitting at least one of the non-speckle illumination light and the speckle illumination light into a fiber bundle with a plurality of optical fibers formed in a bundle.

In the illumination light transmission method related with the present disclosure, for the speckle-enhanced image, at least one of images of a fluid and a flow path may be mentioned.

In the illumination light transmission method related with the present disclosure, for the bright-field image, an organ image may be mentioned and, for the speckle-enhanced image, a blood-flow image may be mentioned.

### [Advantageous Effects of Invention]

According to the present disclosure, the size of the apparatus can be reduced and the work environment of surgical operations can be improved as compared with those of the related-art technologies.

It should be noted that the effects herein are not necessarily restricted thereto; namely, any of the effects herein are valid.

### [Brief Description of Drawings]

[FIG. 1]

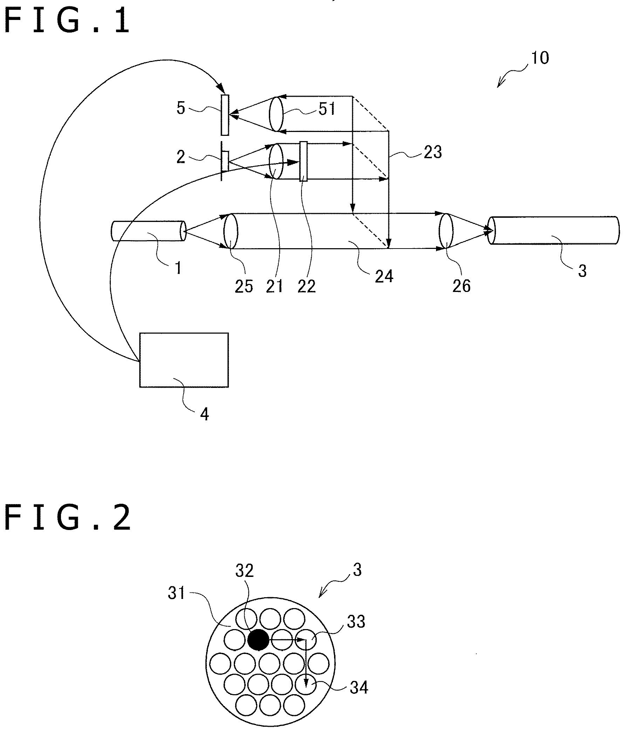

   FIG. 1 is a schematic diagram illustrating a configuration of an illumination light transmission apparatus related with a first embodiment of the present disclosure.
[FIG. 2]
   FIG. 2 is an incident end face view illustrating a fiber bundle.
[FIG. 3]

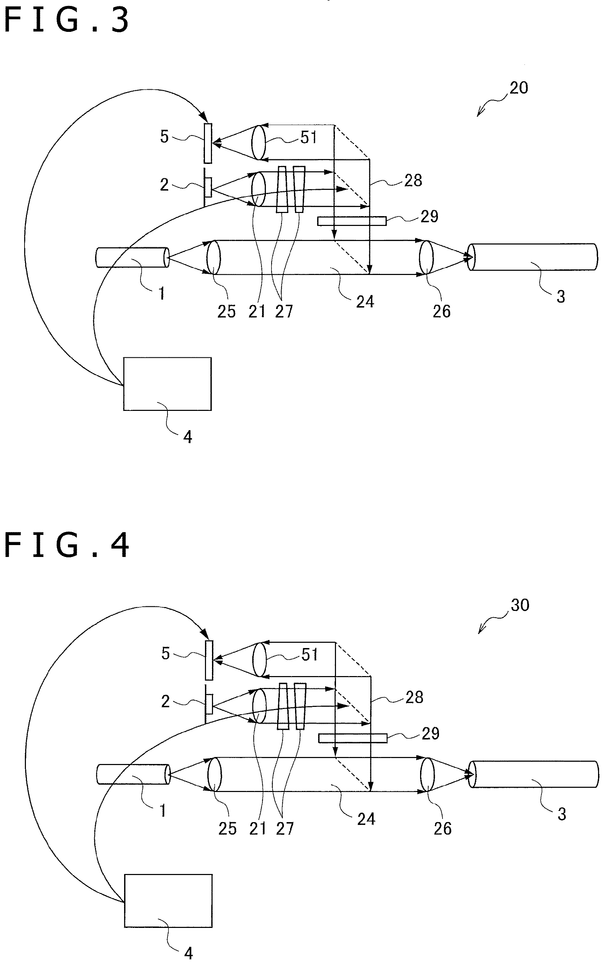

   FIG. 3 is a schematic diagram illustrating a configuration of an illumination light transmission apparatus related with a second embodiment of the present disclosure.
[FIG. 4]
   FIG. 4 is a schematic diagram illustrating a configuration of an illumination light transmission apparatus related with a third embodiment of the present disclosure.
[FIG. 5]

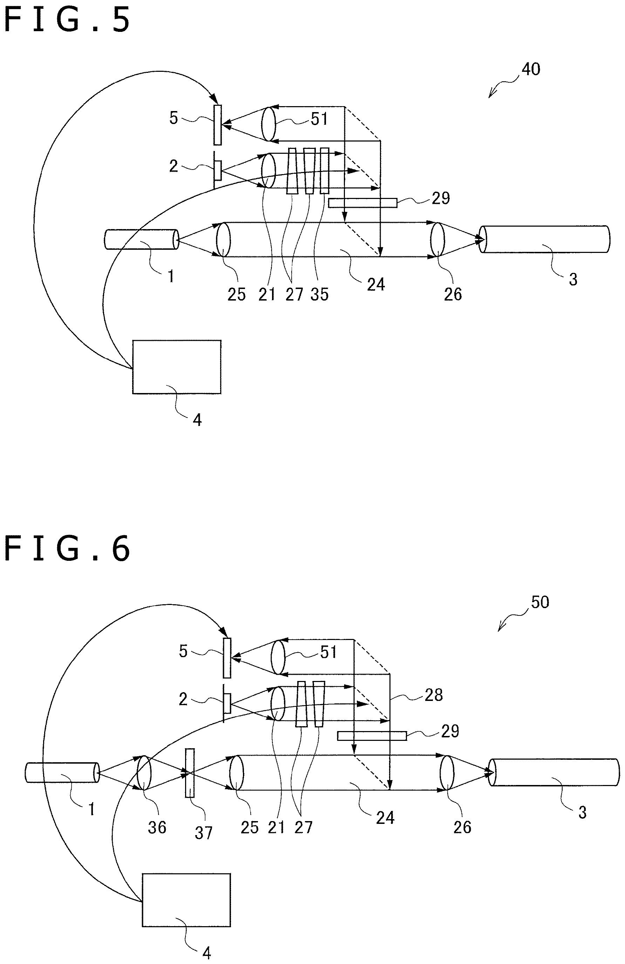

   FIG. 5 is a schematic diagram illustrating a configuration of an illumination light transmission apparatus related with a fourth embodiment of the present disclosure.
[FIG. 6]
   FIG. 6 is a schematic diagram illustrating a configuration of an illumination light transmission apparatus related with a fifth embodiment of the present disclosure.
[FIG. 7]

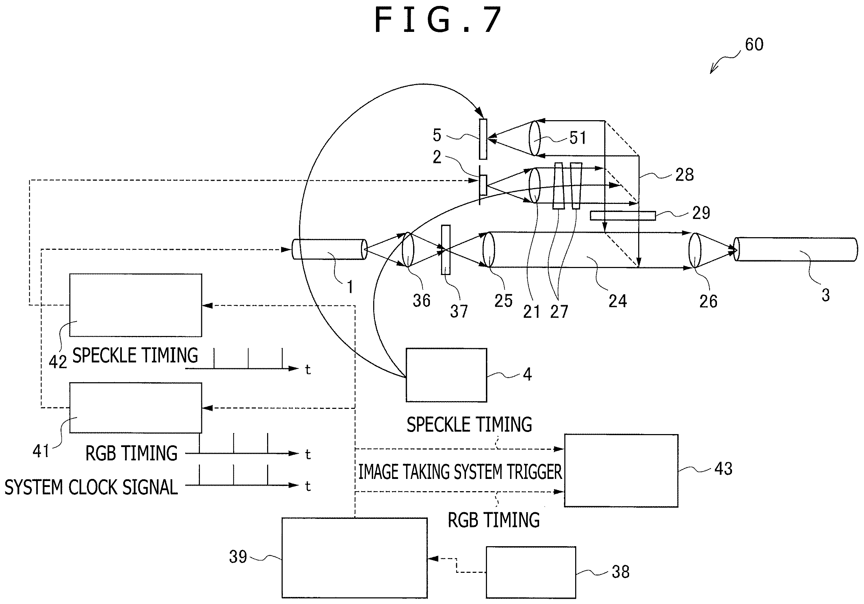

   FIG. 7 is a schematic diagram illustrating a configuration of an illumination light transmission apparatus related with a sixth embodiment of the present disclosure.
[FIG. 8]

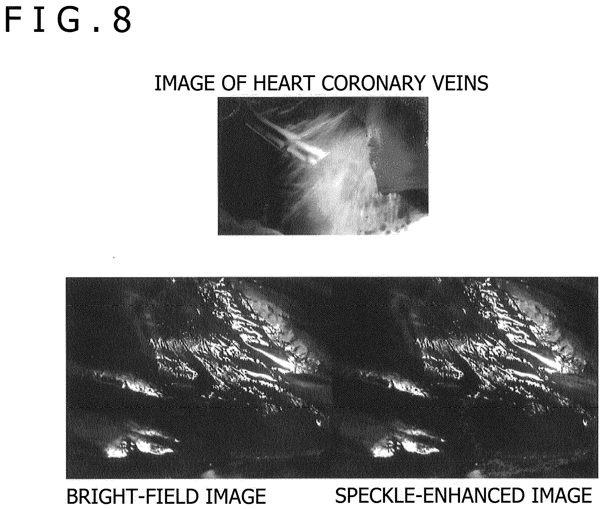

   FIG. 8 includes photographs substituted for drawing that illustrate an image example of a pseudo blood vessel in which pseudo blood flows and an image example of a blood flow of pseudo blood.
[FIG. 9]

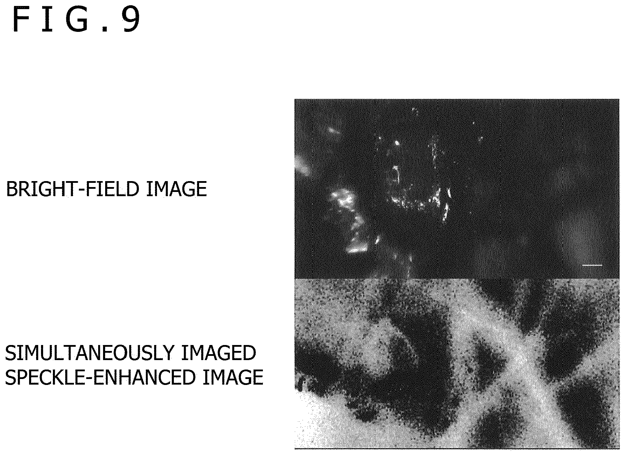

   FIG. 9 includes photographs substituted for drawing that illustrate another image example of a pseudo blood vessel in which pseudo blood flows and another image example of a blood flow of pseudo blood.

### [Description of Embodiments]

The following describes preferred embodiments of the present disclosure with reference to drawings. The preferred embodiments described below are illustrative only and therefore the scope of the present disclosure should not be narrowly interpreted. It should be noted that the description will be done in the following sequence:
First Embodiment (an example of an illumination light transmission apparatus in which a non-speckle illumination light source is configured by a combination of an red-green-blue (RGB) laser beam source and a single-core optical fiber and a light polarization device is arranged on the optical path of a speckle illumination light);
Second Embodiment (an example of an illumination light transmission apparatus in which a non-speckle illumination light source is configured by a combination of a white light-emitting diode (LED) and a fiber bundle and an anamorphic prism or the like is arranged on the optical path of a speckle illumination light);
Third Embodiment (an example of an illumination light transmission apparatus in which a non-speckle illumination light source is configured by a combination of an RGB laser beam source and a fiber bundle and an anamorphic prism or the like is arranged on the optical path of a speckle illumination light);
Fourth Embodiment (another example of an illumination light transmission apparatus in which a non-speckle illumination light source is configured by a combination of an RGB laser beam source and a fiber bundle and an anamorphic prism or the like is arranged on the optical path of a speckle illumination light);
Fifth Embodiment (an example of an illumination light transmission apparatus in which a non-speckle illumination light source is configured by a combination of an RGB laser beam source and a fiber bundle, an anamorphic prism or the like is arranged on the optical path of a speckle illumination light, and a diffusion plate is arranged on the optical path of a non-speckle illumination light); and
Sixth Embodiment (an example of an illumination light transmission apparatus in which a non-speckle illumination light source is configured by a combination of an RGB laser beam source and a fiber bundle, an anamorphic prism or the like is arranged on the optical path of a speckle illumination light, a diffusion plate is arranged on the optical path of a non-speckle illumination light, and a signal generation device or a delay generation circuit or the like is further provided).

### First Embodiment

First, an illumination light transmission apparatus related with the first embodiment of the present disclosure is described.

### (Overall Configuration)

Now, referring to FIG. 1, there is depicted a schematic diagram illustrating the first embodiment of an illumination light transmission apparatus 10 related with the present disclosure. The illumination light transmission apparatus 10 related with the present disclosure mainly has a non-speckle illumination light source 1, a speckle illumination light source 2, a fiber bundle 3, and an optical control block 4. In addition, an image taking system 5 for example may be arranged as required. The following describes details of these components.

### (Non-Speckle Illumination Light Source 1)

With the non-speckle illumination light source 1, the radiation of a non-speckle illumination light is executed. A non-speckle illumination light is a light that does not generate speckles and includes a light of a wavelength range in which light components suitable for illuminating organs for example are distributed. Non-speckle illumination lights include ultraviolet, infrared, and visible lights and include monochromatic lights. The radiation conditions of the non-speckle illumination light source 1 are not especially restricted as long as the effects of the present disclosure are not impaired; for example, radiation angles, radiation positions, and combinations thereof may be set.

The non-speckle illumination light source 1 is used for the acquisition of bright-field images such as the images of organs. A specific structure of the non-speckle illumination light source 1 is not especially restricted as long as the effects of the present disclosure are not infringed; for example, one or more types of known apparatuses or devices enabled to radiate non-speckle illumination lights may be combined without restriction. For example, the non-speckle illumination light source 1 may be configured by a combination of a illumination light source device having an RGB laser beam source capable of radiating a white light mixed by red, green, and blue and a single-core optical fiber. In the illumination light transmission apparatus 10 according to the present disclosure, a white light emitted from a single-core optical fiber is conduced to the fiber bundle 3 through two lenses 25 and 26 (refer to FIG. 1) .

### (Speckle Illumination Light Source 2)

With the speckle illumination light source 2, the radiation of a speckle illumination light is executed. A speckle illumination light is a light that generates speckles and includes a light having a wavelength range in which light components suitable for illuminating flow paths such as blood vessels and fluids such as blood for example are distributed. The radiation conditions of the speckle illumination light source 2 are not especially restricted as long as the effects of the present disclosure are not impaired; for example, radiation angles, radiation positions, and combinations thereof may be set.

The speckle illumination light source 2 is used for the acquisition of speckle-enhanced images. A speckle-enhanced image denotes "an image in which speckles caused by a scattered light obtained from an object are appearing" and includes "an image in which static speckles caused by a scattered light from a standstill object (a flow path such as blood vessels) are appearing" and "an image in which dynamic speckles caused by a scattered light from a moving object (a fluid such as blood) are appearing." For a method of measuring a speckle-enhanced image, a known electronic speckle pattern interferometry (ESPI), a known speckle correlation method, or a known speckle contrast method is available, for example.

The speckle illumination light source 2 may only be a light source capable of radiating a speckle illumination light and therefore not especially restricted to particular light sources as long as the effects of the present disclosure are not impaired; for example, an illumination light source device such as a semiconductor laser (laser diode (LD)) may be used. A speckle illumination light from the speckle illumination light source 2 passes through an aspherical lens 21 and a light polarization device 22 and then is reflected from a mirror 23 to be led into a dichroic mirror 24 from a direction perpendicular to the incident direction of a non-speckle illumination light. The dichroic mirror 24 can be arranged at a position that is related, in Fourier transform, with an emission end face of a single-core optical fiber.

### (Fiber Bundle 3)

The fiber bundle 3 is a bundle of optical fibers. The fiber bundle 3 is arranged at a position optically conjugate with the emission end face of a single-core optical fiber. At an incident end face of the fiber bundle 3, an optical image of a non-speckle illumination light transmitted through a single-core optical fiber and an optical image of a speckle illumination light reflected from the dichroic mirror 24 are formed.

Referring to FIG. 2, there is depicted a diagram illustrating the incident end face of the fiber bundle 3. The image magnification of an optical image of a non-speckle illumination light can be set such that the non-speckle illumination light illuminates all of an end face 31 of the fiber bundle 3. Desirably, the image magnification of an optical image of a speckle illumination light be set such that an incident end face (an incident end face 32 indicated as filled in, for example, in FIG. 2) of only one of the optical fibers is illuminated. This is because the lengths of the optical fibers making up the fiber bundle 3 are not always equal to each other, so that the speckles may be averaged and lowered by optical path difference. However, if there is only one optical fiber that conducts a speckle illumination light, the above-mentioned optical path difference does not occur, so that the speckles can be maintained without being lowered.

It should be noted that the image magnification of an optical image of a speckle illumination light may be set so as to illuminate only a part of each optical fiber. This setup allows speckle-enhanced images obtained at different illumination positions in the fiber bundle 3 to be hour-integrated or averaged, resulting in a reduced spatial granular noise due to speckles.

Some optical fibers constituting the fiber bundle 3 include single-mode optical fibers and multi-mode optical fibers; however, the optical fibers for transmitting a speckle illumination light are preferably single-mode optical fibers from the viewpoint of the maintenance of a speckle pattern without change. This is because, if the optical fibers for transmitting a speckle illumination light are configured by multi-mode optical fibers, the speckle illumination light is diffused into may modes when the fiber bundle 3 bends, thereby possibly changing the speckle pattern. By contrast, if the optical fibers for transmitting a speckle illumination light are configured by single-mode optical fibers, there is only one mode, so that the speckle pattern does not change. Also, speckles may be changed in pattern by the change in polarized wave. In order to prevent this from happening, polarized-wave maintaining optical fibers may be used.

### (Optical Control Block 4)

By the optical control block 4, at least one of the non-speckle illumination light and the speckle illumination light is guided into the fiber bundle 3. "At least one of the non-speckle illumination light and the speckle illumination light is guided into the fiber bundle 3" includes the case in which "both the non-speckle illumination light and the speckle illumination light are guided into the fiber bundle 3," the case in which "only the non-speckle illumination light is guided into the fiber bundle 3" and the case in which "only the speckle illumination light is guided into the fiber bundle 3."

The specific structure of the optical control block 4 is not restricted to any particular structures as long as the effects of the present disclosure are not impaired; for example, one or more types of known circuits or devices may be selected for combination without restriction. For example, a combination of a central processing unit (CPU) and the light polarization device 22 can make up the optical control block 4.

It is also practicable for the optical control block 4 to conduct the speckle illumination light into only one optical fiber selected from among the optical fibers making up the fiber bundle 3. In this case, the selected one optical fiber is preferably a single-mode optical fiber or an optical fiber of which polarized-wave face is maintained, from the viewpoint of maintaining the speckle pattern without change. In addition, it is also practicable for the optical control block 4 to selectively conduct the non-speckle illumination light and the speckle illumination light into the fiber bundle 3.

### (Image Taking System 5)

The image taking system 5 takes an image of a light reflected from the incident end face of the fiber bundle 3 for the observation of an illuminance distribution at the incident end face of the fiber bundle 3.

The method of image taking by the image taking system 5 is not restricted to any particular methods as long as the effects of the present disclosure are not impaired; for example, one or more types of known image taking methods can be selected for combination without restriction. For example, an image taking method based on an imaging device such as a charge coupled device (CCD) sensor or a complementary metal oxide semiconductor (CMOS) sensor may be used.

The specific structure of the image taking system 5 is not restricted to any particular structures as long as the effects of the present disclosure are not impaired; for example, one or more types of known image taking devices or a lens 51 (refer to FIG. 1) may be selected for combination without restriction.

### (Subjects of Illumination)

The illumination light transmission apparatus 10 related with the present disclosure is capable of illuminating a variety of things and is suitable for use in the illumination of subjects that include fluids for example. To be more specific, blood vessels may be mentioned for the subjects of illumination and bloods may be mentioned for fluids. For example, the installation of the illumination light transmission apparatus 10 related with the present disclosure on surgical microscopes or surgical endoscopes allows the surgical operation while making confirmation of the positions of blood vessels.

### (Operations)

The following describes operations of the illumination light transmission apparatus 10 described above.

In the illumination light transmission method according to the present embodiment, a non-speckle illumination light is radiated from the non-speckle illumination light source 1 (a non-speckle illumination light radiation process). Next, at the same time as the radiation of a non-speckle illumination light, a speckle illumination light is radiated from the speckle illumination light source 2 (a speckle illumination light radiation process). It should be noted that, in the speckle illumination light radiation process, the radiation of a speckle illumination light may be executed before the radiation of a non-speckle illumination light or after the radiation of a non-speckle illumination light. Then, both the non-speckle illumination light and the speckle illumination light are emitted into the fiber bundle 3 (an emission process), transmitted through the single fiber bundle 3, and guided into an organ or blood vessels subject to radiation. It should be noted that, in the emission process, only the non-speckle illumination light may be emitted into the fiber bundle 3 or only the speckle illumination light may be emitted into the fiber bundle 3. Next, a light obtained from such a subject of radiation as the organ to which the non-speckle illumination light has been radiated is imaged so as to obtain a bright-field image (an organ image for example) (a bright-field image taking process). Also, a scattered light obtained from such a subject of radiation as blood to which the speckle illumination light has been radiated is imaged so as to obtain a speckle-enhanced image (a blood-flow image for example) (a speckle-enhanced image taking process). It should be noted that, in the bright-field image taking process and the speckle-enhanced image taking process, a bright-field image and a speckle-enhanced image may be alternately taken or a bright-field image and a speckle-enhanced image may be simultaneously taken.

Further, in the present embodiment, the incident end face of the fiber bundle 3 is imaged by the image taking system 5 and, on the basis of results of the imaging, an illuminance distribution at the incident end face of the fiber bundle 3 is observed. In the present embodiment, on the basis of results of this observation, the light polarization device 22 is controlled by the optical control block 4, so that the illumination position of one of the optical fibers or the illumination positions of some of the optical fibers sequentially change. For example, the illumination position (the incident end face 32 in FIG. 2) at which one optical fiber is illuminated sequentially changes to an incident end face 33 and an incident end face 34 depicted in FIG. 2 under the control of the light polarization device 22.

In the present embodiment, after the imaging of subjects of illumination such as organs and blood vessels by speckle illumination lights at the respective illumination positions, speckle-enhanced images obtained at different illumination positions by the fiber bundle 3 are hour-integrated or averaged, resulting in a reduced spatial granular noise due to speckles.

In the present embodiment, since a non-speckle illumination light and a speckle illumination light are transmitted through the single fiber bundle 3, two different illumination optical paths for a non-speckle illumination light and a speckle illumination light need not be arranged. Hence, the apparatus can be reduced in size more than those of related art. It should be noted that the effects described herein are illustrative only and therefore not restricted thereto; for example, other effects may be added.

### Second Embodiment

The following describes an illumination light transmission apparatus related with the second embodiment of the present disclosure. Referring to FIG. 3, there is depicted a schematic diagram illustrating a configuration of the illumination light transmission apparatus related with the second embodiment of the present disclosure. It should be noted that, with reference to FIG. 3, components similar to those of the illumination light transmission apparatus of the first embodiment described before are denoted by the same reference numerals and the description thereof will be skipped.

An illumination light transmission apparatus 20 according to the present embodiment is different from the illumination light transmission apparatus 10 of the first embodiment in that the non-speckle illumination light source 1 is configured by a combination of a white LED and a fiber bundle and in that the lens 21, an anamorphic prism 27, a polarized beam splitter 28, and a *λ*/4 plate 29 are arranged along the optical path of the speckle illumination light.

Since the illumination light transmission apparatus 20 according to the present embodiment has the polarized beam splitter 28, the optical control block 4 can adjust the angle of the polarized beam splitter 28 on the basis of an image obtained by the image taking system 5, thereby providing control to which optical fiber the speckle illumination light is to be coupled at the end face of the fiber bundle 3. It should be noted that the configurations and effects other than those described above in the illumination light transmission apparatus of the present embodiment are the same as those of the first embodiment described above.

### Third Embodiment

The following describes an illumination light transmission apparatus related with the third embodiment of the present disclosure. Referring to FIG. 4, there is depicted a schematic diagram illustrating a configuration of the illumination light transmission apparatus related with the third embodiment of the present disclosure. It should be noted that, with reference to FIG. 4, components similar to those of the illumination light transmission apparatus of the first embodiment described before are denoted by the same reference numerals and the description thereof will be skipped.

An illumination light transmission apparatus 30 according to the present embodiment is different from the illumination light transmission apparatus 10 of the first embodiment in that the non-speckle illumination light source 1 is configured by a combination of an RGB laser beam source and a fiber bundle and in that the lens 21, the anamorphic prism 27, the polarized beam splitter 28, and the *λ*/4 plate 29 are arranged along the optical path of the speckle illumination light.

Since the illumination light transmission apparatus 30 according to the present embodiment has the polarized beam splitter 28, the optical control block 4 can adjust the angle of the polarized beam splitter 28 on the basis of an image obtained by the image taking system 5, thereby providing control to which optical fiber the speckle illumination light is to be coupled at the end face of the fiber bundle 3. It should be noted that the configurations and effects other than those described above in the illumination light transmission apparatus of the present embodiment are the same as those of the first embodiment described above.

### Fourth Embodiment

The following describes an illumination light transmission apparatus related with the fourth embodiment of the present disclosure. Referring to FIG. 5, there is depicted a schematic diagram illustrating a configuration of the illumination light transmission apparatus related with the fourth embodiment of the present disclosure. It should be noted that, with reference to FIG. 5, components similar to those of the illumination light transmission apparatus of the first embodiment described before are denoted by the same reference numerals and the description thereof will be skipped.

An illumination light transmission apparatus 40 according to the present embodiment is different from the illumination light transmission apparatus 10 of the first embodiment in that the non-speckle illumination light source 1 is configured by a combination of an RGB laser beam source and a fiber bundle and in that the lens 21, the anamorphic prism 27, a light polarization device 35 for electronic and optical polarization like a spatial light modulation device or an acousto-optic device, and the *λ*/4 plate 29 are arranged along the optical path of the speckle illumination light.

Since the illumination light transmission apparatus 40 according to the present embodiment has the light polarization device 35, the optical control block 4 can execute light polarization control on the speckle illumination light on the basis of an image obtained by the image taking system 5, thereby providing control to which optical fiber the speckle illumination light is to be coupled at the end face of the fiber bundle 3. It should be noted that the configurations and effects other than those described above in the illumination light transmission apparatus of the present embodiment are the same as those of the first embodiment described above.

### Fifth Embodiment

The following describes an illumination light transmission apparatus related with the fifth embodiment of the present disclosure. Referring to FIG. 6, there is depicted a schematic diagram illustrating a configuration of the illumination light transmission apparatus related with the fifth embodiment of the present disclosure. It should be noted that, with reference to FIG. 6, components similar to those of the illumination light transmission apparatus of the first embodiment described before are denoted by the same reference numerals and the description thereof will be skipped.

An illumination light transmission apparatus 50 according to the present embodiment is different from the illumination light transmission apparatus 10 of the first embodiment in that the non-speckle illumination light source 1 is configured by a combination of an RGB laser beam source and a fiber bundle, in that the lens 21, the anamorphic prism 27, the polarized beam splitter 28, and the *λ*/4 plate 29 are arranged along the optical path of the speckle illumination light, and in that a lens 36 and a diffusion plate 37 for diffusing a non-speckle illumination light are arranged along the optical path of the non-speckle illumination light.

In the illumination light transmission apparatus 50 according to the present embodiment, the diffusion plate 37 is arranged between the lens 36 and the lens 25 along the optical path of a non-speckle illumination light. Then, increasing a numerical aperture allows the radiation of a non-speckle illumination light from the non-speckle illumination light source 1 to the end face of the fiber bundle 3 in a uniform manner. It should be noted that the configurations and effects other than those described above in the illumination light transmission apparatus of the present embodiment are the same as those of the first embodiment described above.

### Sixth Embodiment

The following describes an illumination light transmission apparatus related with the sixth embodiment of the present disclosure. Referring to FIG. 7, there is depicted a schematic diagram illustrating a configuration of the illumination light transmission apparatus related with the sixth embodiment of the present disclosure. It should be noted that, with reference to FIG. 7, components similar to those of the illumination light transmission apparatus of the first embodiment described before are denoted by the same reference numerals and the description thereof will be skipped.

An illumination light transmission apparatus 60 according to the present embodiment is different from the illumination light transmission apparatus 10 of the first embodiment in that the non-speckle illumination light source 1 is configured by a combination of an RGB laser beam source and a fiber bundle, in that the lens 21, the anamorphic prism 27, the polarized beam splitter 28, and the *λ*/4 plate 29 are arranged along the optical path of the speckle illumination light, in that the lens 36 and the diffusion plate 37 are arranged along the optical path of the non-speckle illumination light, and in that a signal generation device 38 for generating a system clock signal, a delay generation circuit 39 for generating a trigger signal for obtaining a bright-field image and a trigger signal for obtaining a speckle-enhanced image, and drivers 41 and 42 for driving the non-speckle illumination light source 1 and the speckle illumination light source 2, respectively, are further added.

In the illumination light transmission apparatus 60 according to the present embodiment, the timings of the trigger signal for obtaining a bright-field image and the trigger signal for obtaining a speckle-enhanced image can be adjusted by the delay generation circuit 39, thereby realizing a so-called field sequential imaging in which a bright-field image and a speckle-enhanced image are sequentially alternately illuminated and imaged.

It should also be noted that the present disclosure can take the following configuration.
(1) An illumination light transmission apparatus including:
   a non-speckle illumination light source configured to radiate a non-speckle illumination light;
   a speckle illumination light source configured to radiate a speckle illumination light;
   a fiber bundle configured by a plurality of optical fibers into a bundle; and
   an optical control block configured to guide at least one of the non-speckle illumination light and the speckle illumination light into the fiber bundle.
(2) The illumination light transmission apparatus according to (1) above, wherein a single-mode optical fiber is included in the optical fibers configuring the fiber bundle.
(3) The illumination light transmission apparatus according to (1) or (2) above, wherein the optical control block guides the speckle illumination light into only one optical fiber selected from the optical fibers configuring the fiber bundle.
(4) The illumination light transmission apparatus according to (3) above, wherein the selected one optical fiber is a single-mode optical fiber.
(5) The illumination light transmission apparatus according to (3) above, wherein the selected one optical fiber is an optical fiber in which a polarized-wave face is maintained.
(6) The illumination light transmission apparatus according to any one of (1) to (5) above, wherein the optical control block selectively guides the non-speckle illumination light and the speckle illumination light into the fiber bundle.
(7) The illumination light transmission apparatus according to any one of (3) to (5) above, wherein the optical control block guides the speckle illumination light into the one selected optical fiber on the basis of an illuminance distribution of the fiber bundle.
(8) The illumination light transmission apparatus according to any one of (1) to (7) above, further including:
   a diffusion plate arranged on an optical path of the non-speckle illumination light so as to diffuse the non-speckle illumination light.
(9) The illumination light transmission apparatus according to any one of (1) to (8) above, wherein the non-speckle illumination light source is used for obtaining a bright-field image and the speckle illumination light source is used for obtaining a speckle-enhanced image.
(10) The illumination light transmission apparatus according to (9) above, wherein the speckle-enhanced image is at least one of images of a fluid and a flow path.
(11) The illumination light transmission apparatus according to (9) or (10) above, wherein the bright-field image is an organ image and the speckle-enhanced image is a blood-flow image.
(12) An illumination light transmission method including:
   a non-speckle illumination light radiation process of radiating a non-speckle illumination light for use in obtaining a bright-field image;
   a speckle illumination light radiation process of radiating a speckle illumination light for use in obtaining a speckle-enhanced image; and
   an emission process of emitting at least one of the non-speckle illumination light and the speckle illumination light into a fiber bundle with a plurality of optical fibers formed in a bundle.
(13) The illumination light transmission method according to (12) above, wherein the speckle-enhanced image is at least one of images of a fluid and a flow path.
(14) The illumination light transmission method according to (12) or (13) above, wherein the bright-field image is an organ image and the speckle-enhanced image is a blood-flow image.

### WORKING EXAMPLES

In the following, advantageous effects of the present disclosure are described in detail in connection with the working examples of the present disclosure. It is to be noted that, in the working examples described below, the numerical aperture (NA) of the lens 21 is 0.5 and the focal length is 30 mm. The numerical aperture (NA) of the lenses 25 and 26 is 0.25, and the focal length is 50 mm. Further, the numerical aperture (NA) of the lens 51 is 0.125, and the focal length is 100 mm. Further, a short-pass dichroic mirror 24 (transmittance 99%) having a cutoff wavelength of 750 nm is installed at a position having a relation of Fourier transform with the emission end face of the fiber bundle 3. Further, for the laser beam source of the speckle illumination light source 2, a semiconductor laser (LD) of a wavelength of 780 nm, a lateral mode of TEM00, a vertical multimode and an output power of 200 mW is used.

### <First Working Example>

In the first working example, an image of a coronary vein of a pig heart is taken using the illumination light transmission apparatus 20 of the second embodiment (refer to FIG. 3). In the present working example, non-speckle illumination light emitted from the fiber bundle 1 having a bundle diameter of 100 *µ*m is introduced to the fiber bundle 3 having the equal bundle diameter of 100 *µ*m using the two lenses 25 and 26.

In the present working example, speckle illumination light emitted from the semiconductor laser (LD) is first collimated by the lens 21 and then shaped into a circular beam by the anamorphic prism 27, whereafter it is reflected by the polarized beam splitter 28 (Tp = 100%, Rs = 100%) and is multiplexed with non-speckle illumination light by the dichroic mirror 24. Then, an image of the laser beam source having a magnification of 1.667 times is formed by the lens 26 on the end face of the fiber bundle 3.

Adjustment of the image formation position on the end face of the fiber bundle 3 by the speckle illumination light can be implemented by adjusting the angle of the dichroic mirror 24 for which an angle adjustment mechanism is provided. In the present working example, although most part of the light propagates in the optical fibers, part of the light not in a coupled state is reflected by the end face of the fiber bundle 3. From within the reflected light, 1% is reflected by the dichroic mirror 24, and 50% of the reflected light passes through the polarized beam splitter 28. In the present working example, the imaging lens 51 and the fiber observation imager 5 are installed rearwardly in the optical path. Consequently, it is possible to observe the end face of the fiber bundle 3.

In the present working example, if an image obtained by the fiber observation imager 5 is observed, then it can be confirmed to which optical fiber the speckle illumination light is coupled at the end face of the fiber bundle 3. If the angle of the polarized beam splitter 28 is adjusted on the basis of this information, then it can be controlled to which fiber the speckle illumination light is to be coupled.

### <Second Working Example>

In the second working example, the illumination light transmission apparatus 30 of the third embodiment (refer to FIG. 4) is used to take an image of a coronary vein of a pig heart. The present working example and the first working example are different in the following points 1 and 2. Since the present working example is same in the other part with the first working example, detailed description of the same is omitted herein.
1. In the RGB laser beam source, the RGB lights have wavelength of 645 nm, 532 nm and 450 nm and are multiplexed using polarization from a plurality of emitters, and have independent components of both p polarization and s polarization.
2. From within the RGB laser beams reflected by the end face of the fiber bundle 3, 1% is reflected by the dichroic mirror 24. Since the reflected light has both components of p polarization and s polarization, a fixed rate of the light passes through the polarized beam splitter 28.

### <Third Working Example>

In the third working example, the illumination light transmission apparatus 40 of the fourth embodiment (refer to FIG. 5) is used to take an image of a coronary vein of a pig heart.

In the present working example, the image formation position of the speckle illumination light on the end face of the fiber bundle 3 is adjusted using a spatial light modulator 35. Since the present working example is same in the other part with the first working example, detailed description of the same is omitted herein.

### <Fourth Working Example>

In the fourth working example, the illumination light transmission apparatus 50 of the fifth embodiment (refer to FIG. 6) is used to take an image of a coronary vein of a pig heart. In the present working example, the end face of the fiber bundle 3 is imaged, and using this as a secondary image, the diffusion plate 37, a diffraction device, a hologram optical device and so forth for increasing the numerical aperture are installed at this position. Since the present working example is same in the other part with the first working example, detailed description of the same is omitted herein.

### <Fifth Working Example>

In the fifth working example, the illumination light transmission apparatus 60 of the sixth embodiment (refer to FIG. 7) is used to take an image of a coronary vein of a pig heart. In the present working example, a bright-field image and a speckle-enhanced image are taken sequentially and alternately to perform so-called field sequential image taking. In the present working example, a system clock signal is generated by the signal generation device 38 and outputted to the delay generation circuit 39. Then, the delay generation circuit 39 generates a trigger signal for a bright-field image and a trigger signal for a speckle-enhanced image using the system clock signal.

In the present working example, a trigger timing of the trigger signal for a bright-field image (RGB timing in FIG. 7) is set to a state synchronized with the system clock signal. A trigger timing of the trigger signal for a speckle-enhanced image (speckle timing in FIG. 7) is set to a state delayed by one half period from the system clock signal.

In the present working example, each trigger signal is introduced into the RGB laser driver 41 or the speckle laser driver 42. In the present working example, the RGB laser beam source and the semiconductor laser (LD) individually and alternately emit non-speckle illumination light and speckle illumination light. In the present working example, the RGB laser beam source and the semiconductor laser (LD) emit pulse laser beams at timings shifted by one half period from each other.

It is to be noted that, for the optical system of the present working example, any of the forms described hereinabove in connection with the first working example to the fourth working example can be adopted. Further, the timing signal for allowing non-speckle illumination light and speckle illumination light to be emitted alternately is used also as an image taking system trigger at a camera 43 side, and image taking is performed in synchronism with a timing of illumination of each illumination light.

For example, FIGS. 8 and 9 include an image example of a pseudo blood vessel through which pseudo blood flows and an image example of a blood flow of pseudo blood. As depicted in the image example of FIG. 8, an image of a pig heart is obtained as a bright-field image, and a blood-flow image is obtained as a speckle-enhanced image. As depicted in the speckle-enhanced image, many speckles are confirmed in a region in which the blood is flowing, and the boundary from another region in which the blood is not flowing is clear. As depicted in the image example of FIG. 9, an image of a pig uterus is obtained as a bright-field image, and a blood-flow image is obtained as a speckle-enhanced image. Also in FIG. 9, many speckles are confirmed in a region in which the blood is flowing (in a white region), and the boundary from another region in which the blood is not flowing (in a dark region) is clear similarly.

It is to be noted that, while, in the present working example, a bright-field image and a speckle-enhanced image are alternately switched to perform illumination and image taking, a bright-field image and a speckle-enhanced image may be illuminated and taken simultaneously. Such illumination and image taking are suitable in the illumination light transmission apparatus of the present disclosure when it is desired to view both a bright-field image and a speckle-enhanced image or when a bright-field image and a speckle-enhanced image are overlapped with each other and viewed in this state.

### [Reference Signs List]

1 Non-speckle illumination light source
2 Speckle illumination light source
3 Fiber bundle
4 Optical control block
5 Image taking system
10, 20, 30, 40, 50, 60 Illumination light transmission apparatus
21, 25, 26, 36, 51 Lens
22 Light polarization device
23 Mirror
24 Dichroic mirror
27 Anamorphic prism
28 Polarized beam splitter
29 *λ*/4 plate
31 End face
32, 33, 34 Incident end face
35 Light polarization device
37 Diffusion plate
38 Signal generation device
39 Delay generation circuit
41, 42 Driver
43 Camera

## Claims

1. An illumination light transmission apparatus comprising:
a non-speckle illumination light source configured to radiate a non-speckle illumination light;
a speckle illumination light source configured to radiate a speckle illumination light;
a fiber bundle configured by a plurality of optical fibers into a bundle; and
an optical control block configured to guide at least one of the non-speckle illumination light and the speckle illumination light into the fiber bundle.

2. The illumination light transmission apparatus according to claim 1, wherein a single-mode optical fiber is included in the optical fibers configuring the fiber bundle.

3. The illumination light transmission apparatus according to claim 1, wherein the optical control block guides the speckle illumination light into only one optical fiber selected from the optical fibers configuring the fiber bundle.

4. The illumination light transmission apparatus according to claim 3, wherein the selected one optical fiber is a single-mode optical fiber.

5. The illumination light transmission apparatus according to claim 3, wherein the selected one optical fiber is an optical fiber in which a polarized-wave face is maintained.

6. The illumination light transmission apparatus according to claim 1, wherein the optical control block selectively guides the non-speckle illumination light and the speckle illumination light into the fiber bundle.

7. The illumination light transmission apparatus according to claim 3, wherein the optical control block guides the speckle illumination light into the one selected optical fiber on the basis of an illuminance distribution of the fiber bundle.

8. The illumination light transmission apparatus according to claim 1, further comprising:
a diffusion plate arranged on an optical path of the non-speckle illumination light so as to diffuse the non-speckle illumination light.

9. The illumination light transmission apparatus according to claim 1, wherein the non-speckle illumination light source is used for obtaining a bright-field image and the speckle illumination light source is used for obtaining a speckle-enhanced image.

10. The illumination light transmission apparatus according to claim 9, wherein the speckle-enhanced image is at least one of images of a fluid and a flow path.

11. The illumination light transmission apparatus according to claim 9, wherein the bright-field image is an organ image and the speckle-enhanced image is a blood-flow image.

12. An illumination light transmission method comprising:
a non-speckle illumination light radiation process of radiating a non-speckle illumination light for use in obtaining a bright-field image;
a speckle illumination light radiation process of radiating a speckle illumination light for use in obtaining a speckle-enhanced image; and
an emission process of emitting at least one of the non-speckle illumination light and the speckle illumination light into a fiber bundle with a plurality of optical fibers formed in a bundle.

13. The illumination light transmission method according to claim 12, wherein the speckle-enhanced image is at least one of images of a fluid and a flow path.

14. The illumination light transmission method according to claim 12, wherein the bright-field image is an organ image and the speckle-enhanced image is a blood-flow image.
